# EUROPÄISCHE PATENTSCHRIFT

(11) **EP 0 430 159 B1**
(45) Veröffentlichungstag und Bekanntmachung des Hinweises auf die Patenterteilung: **09.03.1994**
(21) Anmeldenummer: 90122638.1
(22) Anmeldetag: 27.11.1990
(51) Int. Cl.: A61M 5/32, A61M 5/50

(54) **Sicherheitseinweg-Injektionsspritze**
Single use security injection syringe
Seringue d'injection de securité à usage unique

(30) Priorität: 28.11.1989 DE 3939201
(43) Veröffentlichungstag der Anmeldung: 05.06.1991
(73) Patentinhaber: Odenthal, Jörg Christoph, 30657 Hannover (DE)
(72) Erfinder: Odenthal, Jörg Christoph, 30657 Hannover (DE)
(74) Vertreter: König, Norbert, Dipl.-Phys. Dr.

(56) Entgegenhaltungen:
- EP-A- 0 321 903
- WO-A-88/08315
- DE-A- 3 805 567
- US-A- 4 932 939

## Beschreibung

Die Erfindung betrifft eine Sicherheitseinweg-Injektionsspritze gemäß Oberbegriff des Anspruchs 1.

Handelsübliche Kanülen für medizinische Spritzen und Punktionsgeräte sind mit einem Konus versehen, der das Aufstecken auf die Spritze ermöglicht.

Um eine weitgehende Keimfreiheit einer medizinischen Spritze zu garantieren, werden in zunehmendem Maße nicht wiederverwendbare, sogenannte Einwegspritzen, benutzt. Die Teile werden getrennt verpackt geliefert. Dabei ist die Kanüle in einem ihrer Form angepaßten hülsenförmigen Schutzköcher angeordnet. Zum Gebrauch wird die Kanüle herausgezogen und mit ihrem Konus auf einen Steckansatz des zylindrischen Spritzenkörpers gesteckt. Nach dem Gebrauch wird die Kanüle entweder wieder in die Schutzhülse geschoben oder ohne Schutzhülse weggeworfen.

Das Einfädeln in die Schutzhülse erfordert einen Moment der Aufmerksamkeit, eine ruhige Hand, ein gutes Auge sowie richtige Beleuchtung. Mangelt es hierbei, wird häufig die Einstecköffnung verfehlt. Es kommt dann leicht zu einer Stichverletzung mit der Gefahr der Übertragung von Krankheitserregern. Aus Bequemlichkeitsgründen werden deshalb auch viele Kanülen vorschriftswidrig ohne Schutzhülse in den Abfall geworfen. Die Spritze sowie die Kanüle konnten jedoch bei noch so sorgfältiger Entsorgung immer noch durch dafür nicht geeignete Personen wieder in Benutzung gelangen.

Zur Vermeidung von Verletzungen und zur Vermeidung einer Wiederbenutzung von Kanülen und Injektionsspritzen sind bereits Einweg-Injektionsspritzen vorgeschlagen worden, bei denen die Kanüle nach Gebrauch in das Innere der Spritze hineingezogen werden kann, vgl. US-A-4 838 870, DE-A-3 805 567 und DE-U-8 529 315. Die bekannten Spritzen sind aber konstruktiv relativ aufwendig und teilweise in der Handhabung kompliziert und nicht sicher. Bei der Spritze nach der DE-U-8 529 315 ist die Kanüle nicht vom Spritzenzylinder trennbar.

Die Aufgabe der vorliegenden Erfindung besteht darin, eine Sicherheitseinweg-Injektionsspritze der eingangs genannten Art so zu verbessern, daß die Herstellung und Handhabung einfacher und die Handhabung und Entsorgung sicherer werden, ohne die üblichen Abmessungen verändern zu müssen.

Diese Aufgabe wird durch die Ausbildung gemäß Kennzeichen des Anspruchs 1 gelöst.

Die erfindungsgemäße Sicherheitseinweg-Injektionsspritze weist einen relativ einfachen Aufbau auf und ist daher einfach mit geringen Kosten herstellbar. Die erfindungsgemäße Ausbildung ermöglicht ein völlig unkompliziertes und völlig sicheres Einziehen der Kanüle und damit eine sichere Entsorgung nach Gebrauch der Injektionsspritze.

Vorteilhafte und zweckmäßige Weiterbildungen der erfindungsgemäßen Aufgabenlösung sind in den Unteransprüchen gekennzeichnet.

Die Möglichkeit einer Wiederverwendung wird gänzlich ausgeschlossen durch die Weiterbildung gemäß Anspruch 6. Hierdurch wird auch absolut verhindert, daß durch unbeabsichtigtes Einschieben des Kolbens die Kanüle wieder ungesichert hervortreten kann.

Die Erfindung soll nachfolgend anhand der beigefügten Zeichnungen näher erläutert werden.

Es zeigen:
- Fig. 1: eine Sicherheitseinweg-Injektionsspritze schematisch im Schnitt mit aufgesteckter, exzentrisch angeordneter Kanüle,
- Fig. 2: die Injektionsspritze gemäß Fig. 1 mit eingerasteter Kanüle,
- Fig. 3: die Injektionsspritze nach Fig. 1 oder Fig. 2 mit eingerasteter und eingezogener Kanüle,
- Fig. 4: die bei der Injektionsspritze nach den Fig. 1 bis 3 verwendete Kanüle im Schnitt,
- Fig. 5: eine Rückansicht der Kanüle gemäß Fig. 4,
- Fig. 6: bis 10 verschiedene Ausführungsformen von bei der Sicherheitseinweg-Injektionsspritze nach den Fig. 1 bis 5 verwendeten scheibenförmigen Vorsätzen des Spritzenkolbens zur Aufnahme des Mitnehmers des Spritzenkolbens.

Die Zeichnung zeigt eine Sicherheitseinweg-Injektionsspritze mit einer eine konische Kanülenhalterung 2 aufweisenden Kanüle 3, einem Spritzenzylinder 12, in dem ein Spritzenkolben 4 verschieblich angeordnet ist. Der Spritzenzylinder 12 weist einen konischen Steckansatz 1 für die Kanülenhalterung 2 der Kanüle 3 auf. Im aufgesetzten Zustand ragt die Kanüle 3 in das Innere des Spritzenzylinders 12 durch den Steckansatz 1 hindurch. Am hinteren Ende weist die Kanüle 3 einen Mitnehmer 7 auf, beispielsweise in Form eines Ringflansches oder in Form zweier diametral gegenüberliegender Ansätze.

Der Spritzenkolben ist mit einer Kolbenstange 13 versehen, die mit einer Sollbruchstelle 10 ausgestattet ist.

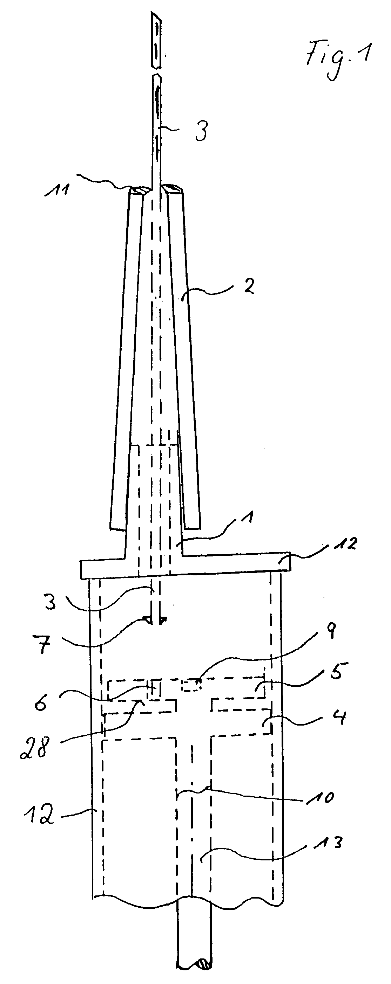

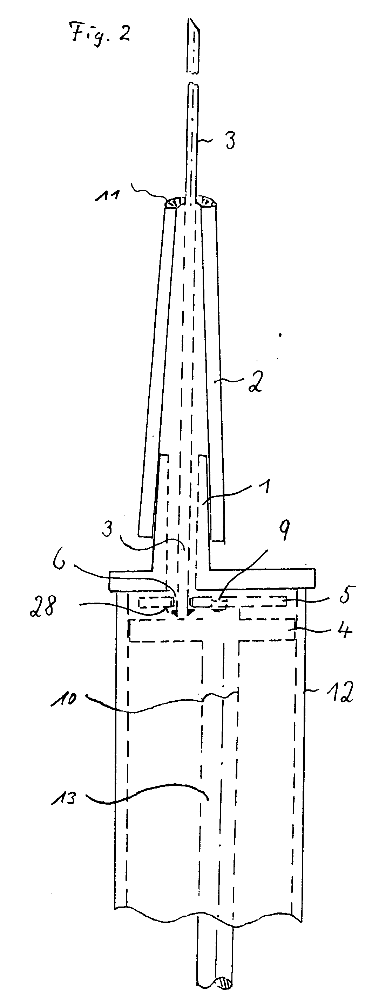

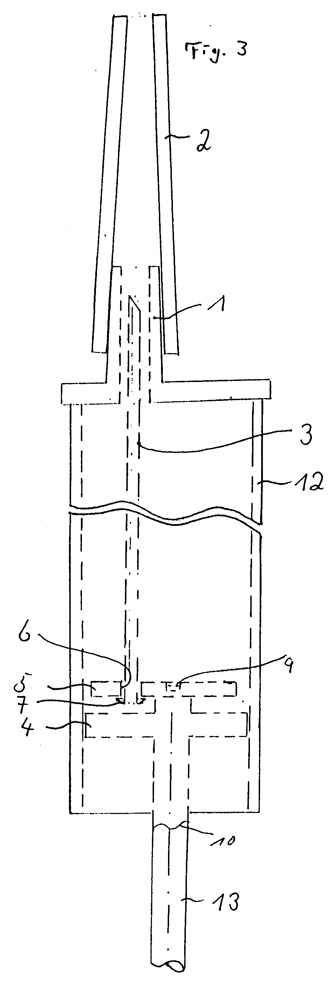

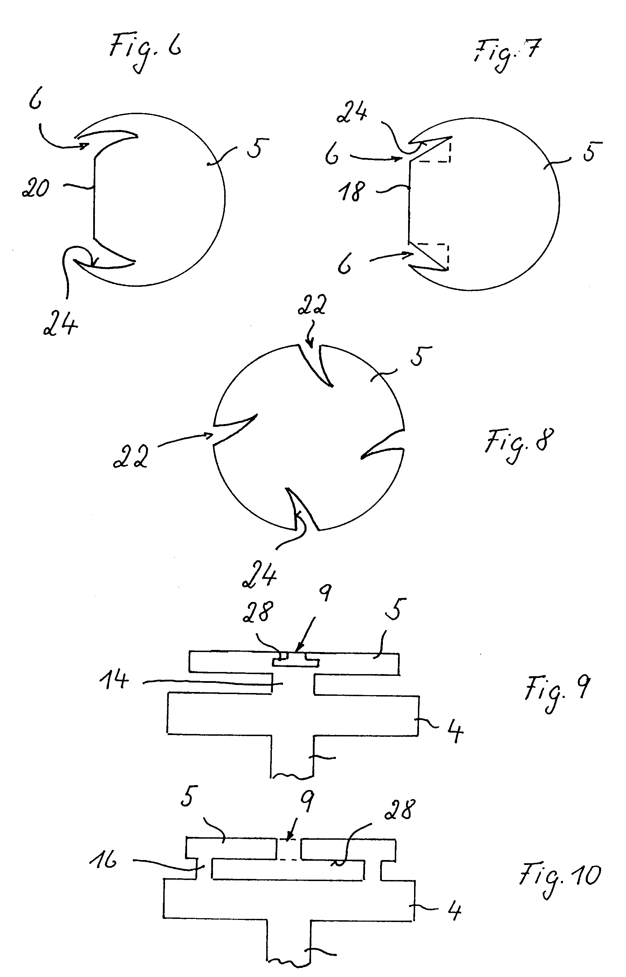

Der Spritzenkolben 4 weist einen scheibenförmigen Vorsatz 5 auf, der einen geringeren Durchmesser aufweisen kann als der Kolben 4 und in dem eine Aufnahmeeinrichtung 6, 9 ausgebildet ist zur Aufnahme des Mitnehmers 7 der Kanüle 3 in der vordersten eingeschobenen Stellung des Spritzenkolbens 4, wie dies in der Fig. 2 dargestellt ist. Zum leichteren Einführen kann der Mitnehmer abgeschrägt sein, vgl. Fig. 1 - 4.

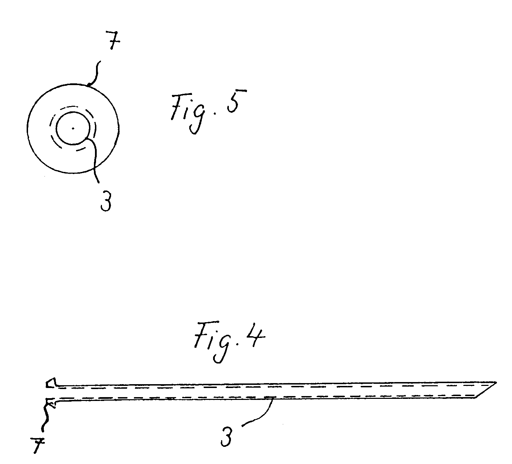

Die Aufnahmeeinrichtung ist in Form von hinterschnittenen Schlitzen 6 ausgebildet, so daß bei Drehung des Spritzenkolbens 4 der Mitnehmer 7 hinter die Hinterschneidung 28 eines Schlitzes greift, derart, daß beim anschließenden Zurückziehen des Spritzenkolbens 4 die Kanüle 3 in den Spritzenzylinder 12 eingezogen wird, wie dies in der Fig. 3 dargestellt ist.

Der scheibenförmige Vorsatz 5 ist mit Abstand zum Spritzenkolben 4 angeordnet vermittels eines zentralen Steges 14 oder mehrerer ringförmig angeordneter Stege 16, vgl. die Fig. 9 und 10.

Die Zeichnung zeigt eine zur Achse des Spritzenzylinders 12 exzentrisch angeordnete Kanüle 3. Bei einer solchen Ausführungsform befinden sich die Schlitze 6 in einer Randausnehmung des scheibenförmigen Vorsatzes 5, wie dies in den Fig. 6 bis 8 dargestellt ist. Die Randausnehmung kann dabei ein Kreisabschnitt 18 oder eine rechteckförmige Ausnehmung 20 sein, vgl. Fig. 7 bzw. Fig. 6. Es können auch mehrere keilförmige Randausnehmungen 22 vorgesehen werden, wie dies die Fig. 8 zeigt.

Die Schlitze 6 befinden sich vorzugsweise im seitlichen Bereich der Ausnehmungen 18 und 20. Sie sind vorzugsweise bogenförmig ausgebildet und verjüngen sich keilförmig. Die Schlitze sind ferner vorzugsweise mit einer als Einlaufflanke für die Kanüle 3 ausgebildeten Schlitzwand 24 versehen.

Anders als dargestellt kann die Kanüle auch zentral, also mit der Achse des Spritzenzylinders 12 ausgerichtet, angeordnet sein. In diesem Falle sind die Schlitze 9 mit Hinterschneidung 28 im Zentrum des scheibenförmigen Vorsatzes 5 ausgebildet, wie dies in den Fig. 9 und 10 dargestellt ist.

Die Kanüle 3 ist am der Kanülenspitze zugewandten Ende der Kanülenhalterung 2 über eine mit einem Reißfaden versehene Manschette 11 lösbar mit der Kanülenhalterung 2 verbunden, vgl. Fig. 1 und 2.

Die Handhabung der Sicherheitseinweg-Injektionsspritze ist wie folgt.

Beim Aufziehen der Spritze wird der Kolben zunächst nach vorn gedrückt und dann zurückgezogen, wodurch sich der Spritzenzylinder füllt. Danach erfolgt der eigentliche Spritzvorgang, wobei der Spritzenkolben 4 mit Hilfe der Kolbenstange 13 nach vorn geschoben wird. In der vordersten Stellung schiebt sich dabei der scheibenförmige Vorsatz 5 über den Mitnehmer 7 der Kanüle 3. Wenn jetzt der Spritzenkolben 4 gedreht wird, schiebt sich der Mitnehmer 7 in einen Schlitz 6 hinein, vgl. Fig. 2 in Verbindung mit den Fig. 6 bis 8. Der Mitnehmer greift dabei hinter die Hinterschneidung des Schlitzes 6.

Danach wird die Kanüle 3 durch Entfernen des Reißfadens von der Manschette 11 und damit von der Kanülenhalterung 2 gelöst. Der Kolben kann jetzt zurückgezogen werden und zieht dabei die Kanüle in das Innere des Spritzenzylinders 12, vgl. Fig. 3.

Die aus dem Spritzenzylinder herausragende Kolbenstange 13 wird an der Sollbruchstelle 10 abgebrochen, so daß eine erneute Benutzung der Injektonsspritze unmöglich ist.

## Patentansprüche

1. Sicherheitseinweg-Injektionsspritze mit einer Kanüle, einer konischen Kanülenhalterung, einem Spritzenzylinder, in dem ein Spritzenkolben verschieblich angeordnet ist und der einen konischen Steckansatz für die Kanülenhalterung aufweist, wobei die Kanüle im aufgesetzten Zustand durch den Steckansatz hindurch in das Innere des Spritzenzylinders ragt und am hinteren Ende einen Mitnehmer aufweist, **da****durch gekennzeichnet,** daß der Spritzenkolben (4) einen scheibenförmigen Vorsatz (5) aufweist, in dem hinterschnittene Schlitze (6; 9) ausgebildet sind, wobei der Mitnehmer (7) der Kanüle (3, 8) in der vordersten eingeschobenen Stellung des Spritzenkolbens (4) von einem der Schlitze aufgenommen wird und hinter dessen Hinterschneidung bei Drehung des Spritzenkolbens (4) greift.

2. Sicherheitseinweg-Injektionsspritze nach Anspruch 1, **dadurch gekennzeichnet,** daß die Kanüle (3) exzentrisch zur Achse des Spritzenzylinders (12) angeordnet ist und die Schlitze (6) in einer Randausnehmung (20) des scheibenförmigen Vorsatzes (5) ausgebildet sind.

3. Sicherheitseinweg-Injektionsspritze nach Anspruch 1, **dadurch gekennzeichnet,** daß die Kanüle (3) mit der Achse des Spritzenzylinders ausgerichtet ist und die Schlitze (9) im Zentrum des scheibenförmigen Vorsatzes (5) ausgebildet sind.

4. Sicherheitseinweg-Injektionsspritze nach Anspruch 2 oder 3, **dadurch gekennzeichnet**, daß der scheibenförmige Vorsatz (5) mit Abstand zum Spritzenkolben (4) angeordnet ist.

5. Sicherheitseinweg-Injektionsspritze nach einem der vorhergehenden Ansprüche, **dadurch gekennzeichnet,** daß die Kanüle (3) am der Kanülenspitze zugewandten Ende der Kanülenhalterung (2) über eine mit einem Reißfaden versehene Manschette (11) lösbar mit der Kanülenhalterung (12) verbunden ist.

6. Sicherheitseinweg-Injektionsspritze nach einem der vorhergehenden Ansprüche, **dadurch gekennzeichnet,** daß der Spritzenkolben (4) eine mit einer Sollbruchstelle (10) ausgestattete Kolbenstange (13) aufweist zum Abbrechen des aus dem Spritzenzylinder (12) herausragenden Teiles der Kolbenstange bei eingezogener Kanüle (3).

7. Sicherheitseinweg-Injektionsspritze nach einem der vorhergehenden Ansprüche, **dadurch gekennzeichnet,** daß die Kanülenhalterung (2) und der Spritzenzylinder (12) mit Markierungen zur gegenseitigen Zuordnung versehen sind.

8. Sicherheitseinweg-Injektionsspritze nach Anspruch 2, **dadurch gekennzeichnet,** daß die Randausnehmung ein Kreisabschnitt (18) oder eine rechteckförmige Ausnehmung (20) ist.

9. Sicherheitseinweg-Injektionsspritze nach Anspruch 2, **dadurch gekennzeichnet,** daß mehrere keilförmige Randausnehmungen (22) vorgesehen sind.

10. Sicherheitseinweg-Injektionsspritze nach Anspruch 8, **dadurch gekennzeichnet,** daß die Schlitze (6) in seitlichen Bereichen der Ausnehmung (18, 20) ausgebildet sind.

11. Sicherheitseinweg-Injektionsspritze nach Anspruch 10, **dadurch gekennzeichnet,** daß die Schlitze (6) bogenförmig (22) ausgebildet sind.

12. Sicherheitseinweg-Injektionsspritze nach Anspruch 10 oder 11, **dadurch gekennzeichnet,** daß die Schlitze (6) zum Schlitzgrund verjüngt ausgebildet sind.

13. Sicherheitseinweg-Injektionsspritze nach Anspruch 1, 3 oder 4, **dadurch gekennzeichnet,** daß der scheibenförmige Vorsatz (5) einen geringeren Durchmessr als der Spritzenkolben (4) aufweist.

14. Sicherheitseinweg-Injektionsspritze nach einem der Ansprüche 1, 3, 4 oder 13, **dadurch gekennzeichnet,** daß der scheibenförmige Vorsatz (5) über einen zentralen Steg (14) oder mehrere ringförmig angeordnete Stege (16) mit dem Spritzenkolben (4) verbunden ist.

15. Sicherheitseinweg-Injektionsspritze nach einem der vorhergehenden Ansprüche, **dadurch gekennzeichnet,** daß die Schlitze (6, 22) eine als Einlauffläche für die Kanüle (3) ausgebildete Schlitzwand (24) aufweisen.

## Claims

1. A safety disposable hypodermic syringe having a cannula, a conical cannula holder, a barrel, in which a plunger is displaceably disposed and which comprises a conical insertable projection for the cannula holder, whereby when fitted the canula extends through insertable projection into the interior of the barrel and at the rear end comprises a catch,
**characterised in that** the plunger (4) comprises a disc**-** shaped attachment (5), in which undercut slits (6; 9) are constructed, whereby in the most forward position of the plunger (4) the catch (7) on the cannula (3, 8) is housed by one of the slits and engages behind its undercut portion during the rotation of the plunger (4).

2. A safety disposable hypodermic needle according to Claim 1,
**characterised in that** the cannula (3) is disposed eccentrically to the axis of the barrel (12) and the slits (6) are constructed in an edge recess (20) in the disc-shaped attachment (5).

3. A safety disposable hypodermic needle according to Claim 1,
**characterised in that** the cannula (3) is aligned with the axis of the barrel and the slits (9) are constructed in the centre of the disc-shaped attachment (5).

4. A safety disposable hypodermic needle according to Claim 2 or 3,
**characterised in that** the disc-shaped attachment (5) is disposed at a distance from the plunger (4).

5. A safety disposable hypodermic needle according to one of the preceding Claims,
**characterised in that** the cannula (3) is detachably connected to the cannula holder (12) at the end of the cannula holder (2) close to the cannula point via a collar (11) provided with a tear-off thread.

6. A safety disposable hypodermic needle according to one of the preceding Claims,
**characterised in that** the plunger (4) comprises a plunger rod (13) equipped with a predetermined breaking point (10) in order to break off the part of the plunger protruding from the barrel (12) when the cannula (3) has been inserted.

7. A safety disposable hypodermic needle according to one of the preceding Claims,
**characterised in that** the cannula holder (2) and the barrel (12) are provided with markings for their mutual coordination.

8. A safety disposable hypodermic needle according to Claim 2,
**characterised in that** the edge recess is a segment of a circle (18) or a rectangular recess (20).

9. A safety disposable hypodermic needle according to Claim 2,
**characterised in that** several wedge-shaped edge recesses (22) are provided.

10. A safety disposable hypodermic needle according to Claim 8,
**characterised in that** the slits (6) are constructed in lateral regions of the recess (18, 20).

11. A safety disposable hypodermic needle according to Claim 10,
**characterised in that** the slits (6) are constructed so that they are curved (22).

12. A safety disposable hypodermic needle according to Claim 10 or 11,
**characterised in that** the slits (6) are constructed so that they taper to the base of the slit.

13. A safety disposable hypodermic needle according to Claim 1, 3 or 4,
**characterised in that** the disc-shaped attachment (5) comprises a smaller diameter than the plunger (4).

14. A safety disposable hypodermic needle according to one of Claims 1, 3, 4 or 13,
**characterised in that** the disc-shaped attachment (5) is connected to the plunger (4) via a central bridge (14) or several bridges disposed in a circle (16).

15. A safety disposable hypodermic syringe according to one of the preceding Claims,
**characterised in that** the slits (6, 22) comprise a slit wall (24) designed as a guide face for the cannula (3).

## Revendications

1. Seringue d'injection de sécurité à usage unique avec une canule, une attache de canule conique, un cylindre de seringue dans lequel un piston de seringue est disposé à coulissement et qui présente une saillie d'enfoncement conique pour l'attache de canule, la canule faisant saillie à travers la saillie d'enfoncement dans l'intérieur du cylindre de seringue à l'état monté et présentant un entraîneur à l'extrémité arrière,
caractérisée en ce que le piston de seringue (4) présente une garniture avant (5) en forme de disque dans laquelle sont formées des rainures en contre-dépouille (6; 9), l'entraîneur (7) de la canule (3, 8) étant reçu, dans la première position d'enfoncement du piston de seringue (4), par l'une des fentes et venant en prise derrière sa contre-dépouille par rotation du piston de seringue (4).

2. Seringue d'injection de sécurité à usage unique selon la revendication 1,
caractérisée en ce que la canule (3) est disposée excentriquement par rapport à l'axe du cylindre de seringue (12) et les fentes (6) sont réalisées dans un évidement de bord (20) de la garniture avant (5) en forme de disque.

3. Seringue d'injection de sécurité à usage unique selon la revendication 1,
caractérisée en ce que la canule (3) est alignée avec l'axe du cylindre de seringue et les fentes (9) sont réalisées au centre de la garniture avant (5) en forme de disque.

4. Seringue d'injection de sécurité à usage unique selon la revendication 2 ou 3,
caractérisée en ce que la garniture avant (5) est disposée à écartement du piston de seringue (4).

5. Seringue d'injection de sécurité à usage unique selon l'une des revendications précédentes,
caractérisée en ce que la canule (3) est reliée de manière démontable à l'attache de canule (2), à l'extrémité de l'attache de canule (2) tournée vers la pointe de canule, par une manchette (11) munie d'un fil d'arrachage.

6. Seringue d'injection de sécurité à usage unique selon l'une des revendications précédentes,
caractérisée en ce que le piston de seringue (4) présente une tige de piston (13) équipée d'une amorce de rupture (10) pour casser la partie de la tige de piston saillant hors du cylindre de seringue (12) lorsque la canule (3) est introduite.

7. Seringue d'injection de sécurité à usage unique selon l'une des revendications précédentes,
caractérisée en ce que l'attache de canule (2) et le cylindre de seringue (12) sont munis de marquages pour la relation réciproque.

8. Seringue d'injection de sécurité à usage unique selon la revendication 2,
caractérisée en ce que l'évidement de bord est une section circulaire (18) ou un évidement rectangulaire (20).

9. Seringue d'injection de sécurité à usage unique selon la revendication 2,
caractérisée en ce que plusieurs évidements de bord (22) en forme de coin sont prévus.

10. Seringue d'injection de sécurité à usage unique selon la revendication 8,
caractérisée en ce que les fentes (6) sont formées dans des zones latérales de l'évidement (18, 20).

11. Seringue d'injection de sécurité à usage unique selon la revendication 10,
caractérisée en ce que les fentes (6) sont réalisées en forme d'arc (22).

12. Seringue d'injection de sécurité à usage unique selon la revendication 10,
caractérisée en ce que les fentes (6) sont réalisées rétrécies vers le fond de fente.

13. Seringue d'injection de sécurité à usage unique selon la revendication 1, 3 ou 4,
caractérisée en ce que la garniture avant (5) en forme de disque présente un diamètre plus faible que le piston de seringue (4).

14. Seringue d'injection de sécurité à usage unique selon l'une des revendications 1, 3, 4 ou 13,
caractérisée en ce que la garniture avant (5) en forme de disque est reliée au piston de seringue (4) par une âme centrale (14) ou plusieurs âmes (16) disposées annulairement.

15. Seringue d'injection de sécurité à usage unique selon l'une des revendications précédentes,
caractérisée en ce que les fentes (6, 22) présentent une paroi de fente (24) réalisée sous forme de surface d'entrée pour la canule (3).
